(19) Europäisches Patentamt
European Patent Office
Office européen des brevets

(11) EP 1 394 262 A1

(12) EUROPEAN PATENT APPLICATION

(43) Date of publication:
03.03.2004 Bulletin 2004/10

(51) Int Cl.[7]: **C12P 35/04**, C12P 35/00, C12P 35/02

(21) Application number: 02078608.3

(22) Date of filing: 30.08.2002

(84) Designated Contracting States:
AT BE BG CH CY CZ DE DK EE ES FI FR GB GR IE IT LI LU MC NL PT SE SK TR
Designated Extension States:
AL LT LV MK RO SI

(71) Applicant: Bioferma Murcia, S.A.
30840 Alhama de Murcia, Murcia (ES)

(72) Inventor: Giesecke, Ulrich Eberhard
82377 Penzberg (DE)

(74) Representative: Davila Baz, Angel et al
c/o Clarke, Modet & Co.,
Goya, 11
28001 Madrid (ES)

# (54) An enzymatic process for preparing beta-lactams

(57) An enzymatic process for preparing β-lactams, by coupling the 7-amino group of a cephalosporin nucleus with the carboxylic function of an acetic acid derivative consisting of a residue with π-electrons, a short spacer and a carboxylic function or derivative. The process comprises using a penicillin amidase or α-amino acid esterase as free enzyme or in any suitable immobilised form; applying no or maximum 10 % organic solvent, applying at least 100 mmol/l cephalosporin nucleus as long as it is soluble, otherwise 50 - 100 % of its maximum solubility is charged, applying the free organic acid in a 3 - 5 fold molar ratio or activated as ester or amide in a 1 - 3 fold molar ratio, and adjusting the pH to 5.0 - 8.0 and the temperature to 0 - 40°C.

EP 1 394 262 A1

## Description

### Field of the invention:

The present invention relates to an enzymatic process for the preparation of semi-synthetic β-lactam antibiotics by reacting a β-lactam precursor with a side chain in free form or activated as an ester or amide as well as recovery of side chain excess and the isolation of the formed product.

### Background of the invention:

β-Lactam antibiotics constitute the most important group of antibiotic compounds, with a long history of clinical use. Among this group the prominent β-lactam antibiotics are the penicillins with a five-membered ring and the cephalosporins with a six-membered ring, which are condensed with a four-membered β-lactam ring. Penicillins consist of the so-called β-lactam nucleus, which is linked through its primary amino group in 6-position to the so-called side chain forming an amide bond. Cephalosporins carry an analogous amide bond in 7-position, but they also vary by different substituents in 3-position.

The conventionally chemical production of semi synthetic β-lactam antibiotics is afflicted with complicated technical requirements and the use of noxious chemicals causing environmental pollution as well as impurities in the product. The synthesis routes are long as it is necessary to introduce protection groups, before modifications can be performed. Since many steps are thus involved, the overall yields are quite low.

There is a great interest for enzymatic routes as alternatives, since enzymatically catalysed reactions are highly selective. Thus the production of many by-products and the effluent and purification problems, which result there from, are reduced or avoided. Furthermore such processes are performed in aqueous environment.

The semi-synthetic routes mostly start from fermentation products such as penicillin G (Pen G), penicillin V (Pen V) and cephalosporin C (Ceph C).

With economical and ecological benefits chemical hydrolysis of penicillin G/V forming the key intermediates 6-aminopenicillanic acid (6-APA) and 7-amino-deacetoxycephalosporanic acid (7-ADCA) was replaced by enzymatic methods using penicillin G/V amidase (PGA or PVA). The other missing key intermediate 7-aminocephalosporanic acid (7-ACA) starting from cephalosporin C is now also on the way to be replaced by a biocatalytical method using a D-amino acid oxidase (DAO) and in a second step a glutaryl-7-ACA acylase (GAC).

Up to now the amide coupling of the β-lactam key intermediates with side chains forming semi synthetic β-lactam antibiotics is done chemically, even though there are efforts for developing enzymatic methods.

These are based mainly on catalysis by penicillin amidase or also called penicillin acylase (EC 3.5.1.11), which can be obtained from *Acetobacter, Achromobacter, Aerobacter, Aerococcus, Aeromonas, Alcaligenes, Aphanocladium, Arthrobacter, Azotobacter, Bacillus, Beneckea, Brevibacterium, Cellulomas, Cephalosporium, Clostridium, Comamonas, Corynebacterium, Erwinia, Escherichia coli, Flavobacterium, Gluconobacter suboxidans, Klebsiella aerogenes, Kluyvera citrophlia, Microbacterium lacticum, Micrococcus, Mycoplana, Norcardia, Paracoccus, Paracolon bacilli, Piocianus, Protaminobacter, Proteus, Pseudobacterium, Pseudomonas, Rhodococus rhodochrous, Rhodopseudomonas, Sacina, Samonella, Serratia, Shigella, Soil bacterium, Spirillum, Staphylococcus, Streptomyces,* Thiobacillus, Xantomonas or Yersinia species [Review: Sudhakaran V. K., Borkar P. S.; Microbial transformation of beta-lactam antibiotics: Enzymes from bacteria, sources and study - a sum up; Hindustan Antibiotics Bulletin 27 (1-4), 63-119; 1985]. Some approaches use α-amino acid esterase (EC 3.1.1.43), which can be obtained from *Acetobacter, Pseudomonas, Streptomyces* species or a *Xantomonas* species [van der Does,T.; An enzymatic process for preparing β-lactam compounds; WO 02/20819 A2; 2000]. The penicillin amidase or the α-amino acid esterase may be used as free enzymes or in any suitable immobilised form.

Penicillin G amidase is normally used for the hydrolysis of Pen G or Ceph G forming the key intermediates (β-lactam nuclei) 6-APA or 7-ADCA for semi synthetic β-lactam antibiotics. It is also a powerful tool for hydrolysis, e. g. of phenylacetyl protected derivatives, or synthesis, e. g. of semi synthetic β-lactam antibiotics with side chains based on acetic acid substituted with an aromatic residue like the natural product phenylacetic acid (PAA). It may be also used for racemic resolution of amino functions:

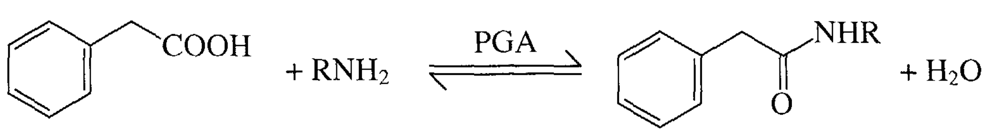

The thermodynamical equilibrium follows the charge state of the acid e.g. PAA. A not charged side chain (e. g. by low pH or adding solvent) is a substrate for synthesis. A dissociated or protonated side chain is no substrate and only hydrolysis takes place. Therefore amino acids like p-hydroxyphenyl glycine or phenylglycine are no substrates for synthesis, since they are at any pH charged:

$$^{+}H_3N\text{-CHR-COOH} \Leftrightarrow {}^{+}H_3N\text{-CHR-COO}^{-} \Leftrightarrow H_2N\text{-CHR-COO}^{-},$$

but no $H_2N$-CHR-COOH

Only in activated form as ester or amide amino acids can be transferred to a not charged form suitable for synthesis:

$$^{+}H_3N\text{-CHR-COOR (not reactive)} \Leftrightarrow H_2N\text{-CHR-COOR (reactive)}$$

$$^{+}H_3N\text{-CHR-CONH}_2\text{ (not reactive)} \Leftrightarrow H_2N\text{-CHR-CONH}_2\text{ (reactive)}$$

Other acids without amino function are only suitable for synthesis at acid pH conditions, since they are there not dissociated. Activated as esters or amides these side chains are at any pH possible substrates. At least reactivity is higher and reaction time shorter due to improved Gibb's energy.

$$R\text{-CH}_2\text{-COOH (reactive)} \Leftrightarrow R\text{-CH}_2\text{-COO}^{-}\text{ (not reactive)}$$

$$R\text{-CH}_2\text{-COOR (reactive)}$$

$$R\text{-CH}_2\text{-CONH}_2\text{ (reactive)}$$

Unfortunately PGA catalyses also hydrolysis of these activated compounds:

$$R\text{-CH}_2\text{-COOR} + H_2O \xrightarrow{PGA} R\text{-CH}_2\text{-COOH} + ROH$$

$$R\text{-CH}_2\text{-CONH}_2 + H_2O \xrightarrow{PGA} R\text{-CH}_2\text{-COOH} + RNH_2$$

The esters or amides are also hydrolysed by the synthesis of the β-lactam antibiotics followed by their hydrolysis, both catalysed by PGA:

$$R\text{-CH}_2\text{-COOR} + H_2N\text{-Nucleus} \xrightarrow{PGA} R\text{-CH}_2\text{-CONH-Nucleus} + ROH$$

$$R\text{-CH}_2\text{-CONH}_2 + H_2N\text{-Nucleus} \xrightarrow{PGA} R\text{-CH}_2\text{-CONH-Nucleus} + NH_3$$

$$R\text{-CH}_2\text{-CONH-Nucleus} + H_2O \xrightarrow{PGA} R\text{-CH}_2\text{-COOH} + H_2N\text{-Nucleus}$$

For the enzymatical β-lactam synthesis it has to be distinguished between the thermodynamically and kinetically controlled approach [Review: Kasche V.; Mechanism and yields in enzyme catalysed equilibrium and kinetically controlled synthesis of β-lactam antibiotics, peptides and other condensation products; Enzyme Mircob. Technol. 8, 4-16; 1986].

In the thermodynamically controlled synthesis free acids are used as side chains. The reaction is slowly, since

low pH values have to be applied, where PGA is not so active. At the end the thermodynamically defined equilibrium is reached, which is conveniently for the stopping criteria. This approach works only with amidases, not with α-amino acid esterase, since this enzyme catalyses only an α-amino acyl transfer. As said before the equilibrium constant follows the charge state of the side chain, which depends on the $k_s$ of the acid and the pH value. The dissociation constant can be shifted to more favourable values by water soluble solvents as ions are less well hydrated. In contrast the $k_s$ is reduced by high ionic strength as ions are better stabilised. Water soluble solvents have also an direct influence on the equilibrium, since they reduce the water activity and therefore water, which is a by-product of synthesis, is apparently removed. Also due to the law of mass action the yield increases by the concentration of β-lactam nucleus and side chain. Temperature and enzyme input have mainly influence on reaction time, but less on yields. It is a common statement, that water soluble solvents increase yields, but our experiments (example 1) confirmed this only for low substrate concentrations. At high substrate input, we surprisingly found, that yields are reduced by water soluble solvents (example 2 & 3).

The kinetically controlled synthesis with esters or amides is much faster, since the Gibb's energy of the activated side chains is improved and more alkaline pH values may be applied, where the amidase or α-amino acid esterase is more active. The higher Gibb's energy is also the reason for yields better than thermodynamic equilibrium. But these condensations have to be terminated just in time, since the formation of β-lactam antibiotics reaches a maximum. The reason is the hydrolysis of activated side chain by hydrolysis or synthesis followed by β-lactam hydrolysis. When all esters or amides are consumed, the conversion will reach at final the thermodynamic equilibrium. At the yield maximum the rate of synthesis from β-lactam nucleus and activated side chain is equal to the rate of hydrolysis of the condensed product. Very often the effects act controversial e. g. a higher pH, higher temperature or more enzyme input increases the activity for hydrolysis and synthesis. In practice a pH around 6 to 7.5 and low temperatures e. g. 10°C are optimal for good yields. For enzyme input a compromise between reaction time and yield has to be defined e. g. 4 - 8 h until the yield maximum is reached works well. Also high substrate concentration will enhance synthesis followed by faster hydrolysis, but better yields will be achieved at higher load. Water soluble solvents and ionic strength will have same effect as discussed before.

The side chain has to have similarities with phenylacetic acid:

- residue with π-electrons (phenyl-, pyridyl-, thienyl-, tetrazolyl-, CN- etc.)
- short spacer ($-CH_2-$, -CHOH-, $CHNH_2-$, $-OCH_2-$, $-SCH_2-$)
- carboxylic function or derivative (-COOH, COOR, CONHR)

The enzymes will convert only dissolved reactants. Therefore their solubility has a great influence on reaction rate and yields.

Downstreaming of the formed product and side chain recovery by simple methods (precipitation or extraction) is a general problem, as the side chain and the formed product have very similar properties in respect to $pK_s$ values and dissolving in organic solvents. Educt and product differ mainly by molar mass. Very often expensive chromatographic separation has to be applied.

For direct 3-processing of a enzymatically synthesised 7-derivative of 7-ACA with thiols in aqueous solution at 50 - 100°C a dark brown colour is formed. Surprisingly this can be avoided, if 7-ACA is removed almost completely, adventurously by salting out the 7-derivative of 7-ACA (if possible).

The history of enzymatic synthesis of β-lactams started already in 1960, as PGA was described and its use for hydrolysis of penicillin G. Also the reverse (thermodynamically controlled) reaction starting from 6-APA and PAA was examined [Rolinson G. N., Batchelor F. R., Butterworth D., Cameron-Wood J., Cole M., Eustace G. C., Hart M. V., Richards M., Chain E. B.; Formation of 6-aminopenicillanic acid from penicillin by enzymatic hydrolysis; Nature 187, 236-237; 1960][Claridge C. A., Gourevitch A., Lein J.; Bacterial penicillin amidase; Nature 187, 237-238; 1960]. The kinetically controlled synthesis was described firstly in 1969, when the amides ($-NH_2$), N-glycine amides ($-NH-CH_2-COOH$), N-hydroxyethyl amides ($-NH-CH_2-CH_2-OH$), thioesters of thioacetic acid ($-S-CH_2-COOH$) and methylesters ($-O-CH_3$) of phenylacetic acid, D-phenylglycine, D/L-mandelic acid, p-hydroxyphenylacetic acid and 3,4-dihydroxyphenylacetic acid were condensed with 6-APA [Cole M.; Factors affecting the synthesis of ampicillin and hydroxypenicillins by the cell-bound penicillin acylase of Escherichia coli; Biochem. J. 115, 759-764; 1969]. Further esters (ethyl, n-/i-propyl, t-butyl and thioglycol) for activation of D-phenylglycine, p-hydroxyphenylglycine, 4-hydroxy-3,5-dichorphenylglycine and cyclohexenylglycine were also condensed with 6-APA [Takahashi, T., Takahashi T., Isono M. (Takeda), Verfahren zur Herstellung von Aminopenicillinen; Deutsche Offenlegungsschrift DT 2 163 792; 1970]. The use of 7-ADCA and 7-ACA was introduced 1971 for the kinetically controlled synthesis with several activated side chains, which were already noted before except 2-thienylglycine methyl ester [Takeshi T., Koichi K., Masao I. (Takeda); Method for the production of cephalosporins; United States Patent US 3,816,253; 1971]. Also 7-amino-3-chloro-3-cephem-4-carboxylic acid was condensed with phenylglycine methylester forming cefaclor [Baldaro E. M.; Effect of temperature on enzymatic synthesis of cephalosporins; Bioorg. Chem. Healtcare Technol., 237-240; 1991]. The only synthesis without aromatic or cyclohexenyl function at the side chain used 225 mM cyanoacetic acid methylester (CNAM) and 18 or 41 mM (12.5 or 5.5 fold) deacetyl-7-ACA (DA-7-ACA) giving 98 or 70 % deacetyl cefacetril [Konecny J., Schneider A., Sieber M.; Kinetics and mechanism of acyl transfer by penicillin acylases; Biotechnol.

Bioeng. 25, 451-467; 1983]. Enzymatic cefacetril synthesis itself was never described before.

The enzymatic synthesis of cephalotin was firstly described 1972. At the thermodynamically controlled synthesis using 20 mM 7-ACA and up to 160 mM thienylacetic acid (ThAH) at pH 6 and 37°C maximum 30 % cephalotin yield were reached. For the kinetically approach using 80 mM thienyl acetyl glycine in a 7 fold faster conversion 50 % yield were obtained [Takahashi, T. T., Kawahara K., Takahashi T. S., Kato K., Yamazaki Y. T. (Takeda); Verfahren zur Herstellung von Cephalosporinen; Deutsche Offenlegungsschrift DT 2 360 265; 1972]. Yields up to 96 % crude cephalotin were described by using immobilised PGA on a column. This was achieved by charging 26-fold thienylacetic acid methylester for the kinetically controlled synthesis, which is of cause a hindrance for economic usage [Fujii T., Shibuya Y. (Toyo Jozo.); Enzymatic production of cephalothin; United States Patent US 3,853,705; 1972]. By activation with low molecular polyethylene glycolesters up to 85 % yield were achieved (18 mM 7-ACA, 78 mM thienylacetic acid tetraethylene glycolester, pH 7 and 37°C using PGA on a column) [Kondo E., Mitsugi T., Fujiwara T., Muneyuki R. (Shionogi); Enzymatisches Verfahren zur Herstellung von ß-Lactamantibiotika und Acylverbindungen zur Durchführung des Verfahrens; Deutsche Offenlegungsschrift DE 3035467 A1; 1979]. At thermodynamically controlled synthesis 95 % cephalotin yield were obtained by charging a high side chain excess (15 mM 7-ACA and 400 mM ThAH), applying > 30 % solvents (examples with 50 - 65 % dioxane, 65 % acetone, 50 % butanediol, 70 % ethanol and 50 - 60 % DMF) and passing the solution through a column with immobilised PGA at 4 - 37°C and a initial pH between 5 and 8 (pH 6 for cephalotin) [Guisan J. M., Fernandez-Lafuente R., Alvaro G., Blanco R. M., Rosell C. M; Method for the synthesis of semi-synthetic antibiotics in thermodynamically controlled water-cosolvent organic miscible apolar systems by using penicillin G acylase; European Patent Application EP 0 458 932 A1; 1989]. The low 7-ACA concentration was caused by its maximum solubility. By applying pH 7.2 and reducing the pH during synthesis to pH 6.4 the 7-ACA input was enhanced to 50 mM in 50 % DMF. While charging only 4-fold ThAH also 95 % cephalotin yield were achieved [Fernandez-Lafuente R., Alvaro G., Blanco R. M., Guisan J. M.; Equilibrium controlled synthesis of cephalothin in water-cosolvent systems by stabilized penicillin G acylase; Appl. Biochem. Biotechnol. 27, 277-290; 1991].

Cefoxitin <it will be possible> is an other product from ThAH. It uses as β-lactam nucleus 3-aminocarbonyloxymethyl-7-methoxy-3-cephem-4-carbonic acid (7-MACA), which surprisingly can be produced analogous to cephalosporin C from cephamycin C using DAO and GAC <to be evaluated>. Neither enzymatic cleavage of cephamycin C to 7-MACA nor consecutive enzymatic synthesis of cefoxitin was ever described before.

Cefazolin maybe formed by firstly 3-processing of 7-ACA with 2-mercapto-5-methyl-1,3,4-thiadiazole (MMTD) forming 7-amino-3-(5-methyl-1,3,4-thiadiazole-5-yl) thiomethyl-3-cephem-4-carboxylic acid (TDA) followed by 7-processing with tetrazolylacetic acid (TzAH) or alternatively by firstly 7-processing of 7-ACA with TzAH forming tetrazolyl-7-ACA (TzA-7-ACA) followed by 3-processing with MMTD. The thermodynamically controlled synthesis is not possible as TzAH is a strong acid, but only uncharged side chains are accepted by PGA as substrate. For the kinetical approach using 6 mM TzAH in form of the methylester (TzAM) with 2 mM 6-APA, 7-ADCA, 7-ACA or 7-amino-3-pyridylmethyl-3-cephem-4-carboxylic acid at pH 6.5 and 37°C up to 80 % yield were noted [Shimizu M., Okachi R., Kimura K., Nara T.; Purification and properties of penicillin acylase from *Kluyvera citrophila;* Agr. Biol. Chem. 39 (8), 1655-1661; 1975]. Up to 55.7 % cefazolin yield were reported starting from tetrazolylacetic acid pentaethylene glycolester and TDA [Muneyuki R., Mitsugi T., Kondo E.; Water-soluble esters: useful enzyme substrates for the synthesis of β-lactam antibiotics; Chem. Industry 7, 159-161; 1981]. By coupling 29 mM TDA with a 1 to 5 fold ratio of various esters at pH 6.8 and 35°C up to 63.8 % cefazolin yield were obtained. The high temperature is obviously chosen in order to dissolve TDA in such concentration at this low pH [Kostadinov M., Nikolov A., Tsoneva N., Petkov N.; New tetrazole-1-acetic acid esters for enzymatic synthesis of cefazolin; Appl. Biochem. Biotechnol. 33, 177-182; 1992]. Applying 50 mM 7-ACA and 150 mM TzAM (3 fold) at pH 6.5 and 4°C 98 % tetrazolyl-7-ACA (TzA-7-ACA) were obtained. Without isolation 3-processing with MMTD to cefazolin was performed at 65°C in 70 % yield [Justiz O. H., Fernandez-Lafuente R., Guisan J. M., Negri P., Pagani G., Pregnolato M., Terreni M.; J. Org. Chem. , 62 (26), 9099-9106 (1997); One-pot chemoenzymic synthesis of 3'-functionalized cephalosporines (cefazolin) by three consecutive biotransformations in fully aqueous medium].

Cefazedon <to be evaluated> has the same substitution in 3-position, but carries 3,5-dichlor-4-pyridon-1-yl-acetic acid (CPH) in 7-position. The reaction sequence starts either via 3-processing of 7-ACA with MMTD forming TDA followed by 7-processing with CPH or via 7-processing of 7-ACA with CPH forming 3,5-dichlor-4-pyridon-1-yl-acetyl-7-ACA (CP-7-ACA) followed by 3-processing with MMTD. No example is described for the unexpected enzymatic condensation of CPH in any activated form with 7-ACA or TDA.

Ceftexol <to be evaluated> has the same substitution as cefazolin in 7-position, but is 3-processed with 2-mercapto-1,3,4-thiadiazol (MTD). The reaction sequence starts either via 3-processing of 7-ACA with MTD forming 3-[[(1,3,4-thiadiazol-2-yl)thio]methyl-7-ACA (TA) followed by 7-processing with TzAM or via 7-processing of 7-ACA with TzAM forming TzA-7-ACA followed by 3-processing with MTD. No example is described for the unexpected enzymatic condensation of TA with TzAM.

Cefamandol has also two substitutions. It maybe formed by firstly 3-processing of 7-ACA with 1-methyl-5-mercaptotetrazole (MMTZ) forming 7-amino-3-(1-methyl-1H-tetrazol-5-yl) thiomethyl-3-cephem-4-carboxylic acid (TZA) followed by 7-processing with D-mandelic acid (MAH) or alternatively by firstly 7-processing with MAH forming mandoyl-7-ACA (MA-7-ACA) followed by 3-processing with MMTZ. MAH in form of its methylester (MAM, 5 fold molar ratio) was condensed with TZA forming only 8 % cefamandol, where the L-enantiomer gave 35 % yield [Fuganti C., Rosell C. M., Rigoni R., Servi S., Tagliani A., Terreni M.; Penicillin acylase mediated synthesis of formyl cefamandole; Biotechnol. Letters 14 (7), 543-546; 1992]. By converting 50 mM 7-ACA with 25 mM MAM at pH 7 and 4°C 25 % MA-7-ACA yield were reported [Fernandez-Lafuente R., Rosell, C. M., Guisan J. M.; The use of stabilised penicillin acylase derivatives improves the design of kinetically controlled synthesis; J. Mol. Catal. A: Chem. 101 (1), 91-97; 1995]. Applying 50 mM 7-ACA and 150 mM MAM (3 fold) at pH 6.5 and 4°C 69 % MA-7-ACA were obtained. In presence of 20 % methanol and 1 M phosphate buffer yield was improved up to 80 %. Without isolation 3-processing with MMTZ to cefamandol was performed at 65°C in 60 % yield [Terreni M., Pagani G., Ubiali D., Fernandez-Lafuente R., Mateo C., Guisan J. M.; Bioorg. Med. Chem. Lett. 11 (18), 2429-2432 (2001); Modulation of penicillin acylase properties via immobilization techniques: one-pot chemoenzymatic synthesis of cephamandole from cephalosporin C]. The thermodynamical approach with free MAH and the kinetically controlled synthesis by activation as hydroxyethylester (MAG, produced from ethylene glycol) was never described before.

Cefoperazon has the same substitution in 3-position, but carries D-p-hydroxyphenylglycine (HPH) in 7-position, which is further modified finally at the introduced aminogroup with 2,3-dioxo-4-ethyl-1-piperazin-carbonylchloride. The reaction sequence starts either via 3-processing of 7-ACA with MMTZ forming TZA followed by 7-processing with HPH to p-hydroxyphenylglycyl-TZA (HP-TZA) or via 7-processing of 7-ACA with HPH forming p-hydroxyphenylglycyl-7-ACA (HP-7-ACA) followed by 3-processing with MMTZ to HP-TZA. The thermodynamically controlled synthesis is not possible as HPH is an amino acid, but only uncharged side chains are accepted by PGA as substrate. As mentioned already before most often the methylester (HPM) is used for activation of HPH. For cefprozil, cefadroxyl and amoxicillin the activation of HPH as hydroxyethylester (HPG, produced from ethylene glycol) charged in approximately a 3 fold ratio is introduced giving 90 - 99 % yield [Usher J. J., Romancik G., Bristol-Meyers Squibb; PCT Int. Appl. WO 98/04732 A1 (1996); Synthesis of β-lactam antibacterials using soluble side chain esters and enzyme acylase]. No example is described for enzymatic condensation of HPH in any activated form with 7-ACA or TZA.

Cefatrizin <it will be possible> is an other product from 7-ACA and HPH. The reaction sequence starts either via 3-processing of 7-ACA with 4-mercapto-1,2,3-triazole (MTZ) forming 3-[[(1,2,3-triazol-4-yl)thio]methyl-7-ACA (MTA) followed by 7-processing with HPH or via 7-processing of 7-ACA with HPH forming HP-7-ACA followed by 3-processing with MTZ. No example is described for enzymatic condensation of HPH in any activated form with (7-ACA or) MTA.

Cefotiam <to be evaluated> is either formed via 3-processing of 7-ACA with 5-mercapto-1-(2-dimethylaminoethyl)-1H-tetrazole (MDTZ) forming 3-[[[1-[2-(dimethylamino)-ethyl]-1-H-tetrazol-5-yl]thiomethyl-7-ACA (DZA) followed by 7-processing with 2-aminothiazolylacetic acid (ATA) or via 7-processing of 7-ACA with ATH forming 2-aminothiazolylacetyl-7-ACA (ATA-7-ACA) followed by 3-processing with MDTZ. No example is described for enzymatic condensation of ATA in any activated form with 7-ACA or DZA as only low yields are obtained. We surprisingly found, that 2-formylamino-4-thiazole acetic acid (FATA) in form of its methyl (MFATA) or ethyl ester (EFATA) is a suitable side chain. The formyl group can easily be removed at acid condition.

Cefapirin <to be evaluated> is formed by 7-processing or 7-ACA with 4-pyridylthioacetic acid (PTH). No example is described for the unexpected enzymatic condensation of PTH in any activated form with 7-ACA.

**Summary of the invention:**

We examined the enzymatic synthesis of cephalosporins using PGA starting from 7-ACA, 3-processed derivatives thereof or 7-MACA.

For the thermodynamically controlled synthesis we surprisingly found, that the benefit by water soluble solvents on the $k_s$ value of the side chain, water activity and conversions turn at higher concentration into its opposite. By that excellent yields can be obtained without charging a solvent, which would be of harm for the PGA. As example, but not limited to this, we describe the synthesis of:

- cephalotin from 7-ACA and ThAH
- mandoyl-7-ACA from 7-ACA and MAH

For the kinetically controlled synthesis we surprisingly found, that activation as hydroxyethylester gives much better yields as activation as the commonly used methylester. As example, but not limited to this, we describe the synthesis of:

- cefamandol from TZA and MAG versus MAM
- mandoyl-7-ACA from 7-ACA and MAG versus MAM

Kinetically controlled synthesis of cefazolin from TDA and TzAH preferably activated as methylester is hindered by the low solubility of TDA at reasonably pH. At higher pH, where TDA is soluble, TzAM is hydrolysed fast by PGA. We could surprisingly overcome this problem by charging preferably 50 - 60 mmol/l TDA, applying a pH gradient and feeding solid TzAM during synthesis.

We found much improved conditions for the kinetically controlled synthesis of TzA-7-ACA from 7-ACA with TzAH preferably activated as methylester.

**Description of the invention:**

Thus, in a first aspect the invention is related with an enzymatic process for coupling the 7-amino group of a cephalosporin nucleus with the carboxylic function of an acetic acid derivative consisting of a:

- residue with π-electrons (phenyl-, pyridyl-, thienyl-, tetrazolyl-, CN- etc.)
- short spacer (-$CH_2$-, -CHOH-, $CHNH_2$-, -$OCH_2$-, -$SCH_2$- etc.)
- carboxylic function or derivative (-COOH, COOR, CONHR),

which is characterised by:

➢ using a penicillin amidase (EC 3.5.1.11 ) or α-amino acid esterase (EC 3.1.1.43) from *Acetobacter, Achromobacter, Aerobacter, Aerococcus, Aeromonas, Alcaligenes, Aphanocladium, Arthrobacter, Azotobacter, Bacillus, Beneckea, Brevibacterium, Cellulomas, Cephalosporium, Clostridium, Comamonas, Corynebacterium, Erwinia, Escherichia coli, Flavobacterium, Gluconobacter suboxidans, Klebsiella aerogenes, Kluyvera citrophlia, Microbacterium lacticum, Micrococcus, Mycoplana, Norcardia, Paracoccus, Paracolon bacilli, Piocianus, Protaminobacter, Proteus, Pseudobacterium, Pseudomonas, Rhodococus rhodochrous, Rhodopseudomonas, Sacina, Samonella, Serratia, Shigella, Soil bacterium, Spirillum, Staphylococcus, Streptomyces, Thiobacillus, Xantomonas* or *Yersinia* species as free enzyme or in any suitable immobilised form,
➢ applying no or maximum 10 % organic solvent,
➢ applying at least 100 mmol/l cephalosporin nucleus as long as it is soluble, otherwise 50 - 100 % of its maximum solubility is charged,
➢ applying the free organic acid in a 3 - 5 fold molar ratio or activated as ester or amide in a 1 - 3 fold molar ratio,
➢ adjusting pH 5.0 - 8.0 and 0 - 40°C.

According to a second aspect, the invention is related with an enzymatic process as defined above, under thermodynamical control, wherein:

➢ no or maximum 10 % organic solvent is used,
➢ at least 100 mmol/l cephalosporin nucleus is applied,
➢ the free organic acid is charged in a 3 - 5 fold molar ratio,
➢ the pH is reduced by acid according the reaction progress from initially pH 7.5 - 6.25 to pH 6.25 - 5.0,
➢ 10 - 40°C is adjusted.

According to a third aspect, the invention is related with an enzymatic process as defined in the first aspect of the invention, under kinetical control, wherein:

➢ no organic solvent is used,
➢ at least 100 mmol/l cephalosporin nucleus is applied as long as it is soluble, otherwise 50 - 100 % of its maximum solubility is charged,
➢ the organic acid is charged as ester or amide in a 1 - 3 fold molar ratio,
➢ pH 6.0 - 8.0 is titrated by alkaline,
➢ 0 - 30°C is adjusted.

As described above, the process of the invention is carried out using the following reaction components:

- the organic acid is ThAH, the cephalosporin nucleus is 7-ACA or 7-MACA and the formed product is cephalotin or cefoxitin.

- the organic acid is D-mandelic acid, the cephalosporin nucleus is 7-ACA and the formed product is mandoyl-7-ACA.

- the organic acid is 3,5-dichlor-4-pyridon-1-yl-acetic acid, the cephalosporin nucleus is 7-ACA or TZA and the formed product is 3,5-dichlor-4-pyridon-1-yl-acetyl-7-ACA or cefazedon.

- the organic acid is 2-formylamino-4-thiazole acetic acid, the cephalosporin nucleus is 7-ACA or DZA and the formed product is n-formylamino-4-thiazole acetyl-7-ACA or n-formyl-cefotiam.

- the organic acid is 4-pyridylthioacetic acid, the cephalosporin nucleus is 7-ACA and the formed product is cefapirin.

- the organic acid is cyanoacetic acid, the cephalosporin nucleus is 7-ACA and the formed product is cefacetril.

- the organic acid is activated as methyl- or ethylester. It is charged in one portion or initially equimolar to the cephalosporin nucleus with further feed during synthesis.

- the organic acid is activated as hydroxyethylester, prepared from 3 - 10 fold ethylene glycol and might be used without further purification.

- The organic acid is D-mandelic acid, the cephalosporin nucleus is 7-ACA or TZA and the formed product is mandoyl-ACA or cefamandol.

- the organic acid is p-hydroxyphenylglycine, the cephalosporin nucleus is 7-ACA, TZA or MTA and the formed product is p-hydroxyphenylglycyl-7-ACA, p-hydroxyphenylglycyl-TZA or cefatrizin.

- the organic acid is TzAH, the cephalosporin nucleus is 7-ACA, TDA or TA and the formed product is TzA-7-ACA, cefazolin or ceftexol.

Thus, we describe the first time the synthesis of:

- cefoxitin from 7-MACA and ThAH as free acid or activated e. g. as methylester
- 3,5-dichlor-4-pyridon-1-yl-acetyl-7-ACA from 7-ACA and 3,5-dichlor-4-pyridon-1-yl-acetic acid as free acid or activated as methyl- or hydroxyethylester
- cefazedon from TZA and 3,5-dichlor-4-pyridon-1-yl-acetic acid as free acid or activated as methyl- or hydroxyethylester
- ceftexol from TA with TzAH preferably activated as methylester
- p-hydroxyphenylglycyl-7-ACA from 7-ACA and HPH preferably activated as hydroxyethylester
- p-hydroxyphenylglycyl-TZA from TZA and HPH preferably activated as hydroxyethylester
- cefatrizin form MTA and HPH preferably activated as hydroxyethylester
- n-formylamino-4-thiazole acetyl-7-ACA from 7-ACA and 2-formylamino-4-thiazole acetic acid as free acid or activated e. g. as ethylester
- n-formyl-cefotiam from DZA and 2-formylamino-4-thiazole acetic acid as free acid or activated e. g. as ethylester
- cefapirin from 7-ACA and 4-pyridylthioacetic acid as free acid or activated as methyl- or hydroxyethylester
- cefacetril from 7-ACA and cyanoacetic acid as free acid or activated e. g. as methylester

We describe the first time a process for 3-processing of a enzymatically synthesised 7-derivative of 7-ACA with a heterocyclic thiol in aqueous solution, where:

- the 7-derivative of 7-ACA is polluted with less than 0.2 % 7-ACA,
- 50 - 250 mmol/l 7-derivative of 7-ACA are charged,
- the heterocyclic thiol is charged at least equimolar to the cephalosporin,
- pH 6 - 8.5 is adjusted,
- 50 - 75°C are applied for 3-processing

In said process the heterocylic thiol is MMTD, the 7-derivative of 7-ACA is TzA-7-ACA and the formed product is cefazolin, where the used TzA-7-ACA is salted out by sodium or potasium salts and preferably by 15-30% NaCl.
We describe the first time the enzymatic cleavage of cephamycin C to 3-aminocarbonyloxymethyl-7-methoxy-3-cephem-4-carbonic acid (7-MACA), DAO and GAC.
For downstreaming it is often a problem to separate the side chain from the formed cephalosporin, as the

chemical properties are very similar. We surprisingly found, that ThAH can be recovered by extraction with toluene or xylene, while cephalotin remains in aqueous solution and can be isolated afterwards by precipitation at acid pH or salting out.

For 3-processing of a enzymatically synthesised 7-derivative of 7-ACA it has to be free of 7-ACA, as 7-ACA causes a dark brown colour formation while treating it with thiols in aqueous solution at 50 - 100°C. Surprisingly some intermediates e. g. TzA-7-ACA can be salted out without interfering 7-ACA pollution.

The following examples are for illustration purposes only and in no way limit the scope of this invention:

**Example 1: influence of water-soluble solvents on the thermodynamically controlled synthesis of cephalotin at low educt concentration:**

The experiments were performed in 400 ml scale with 20 kU/l PGA-450, 50 mmol/l 7-ACA, 200 mmol/l ThAH, 1 mol/l HCl for adjusting the pH according to the first diagram, 20°C and 250 rpm:

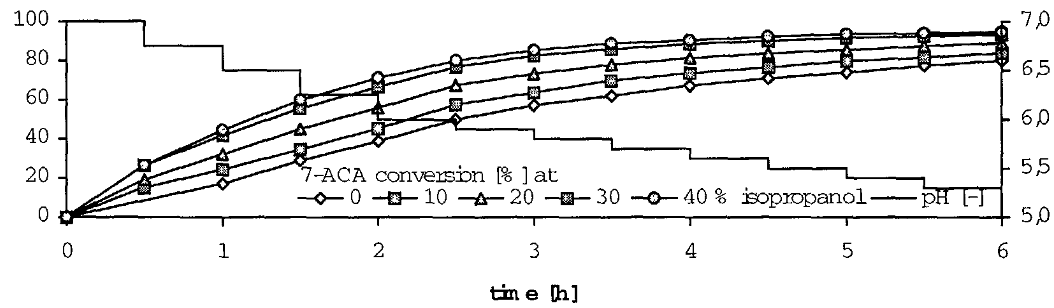


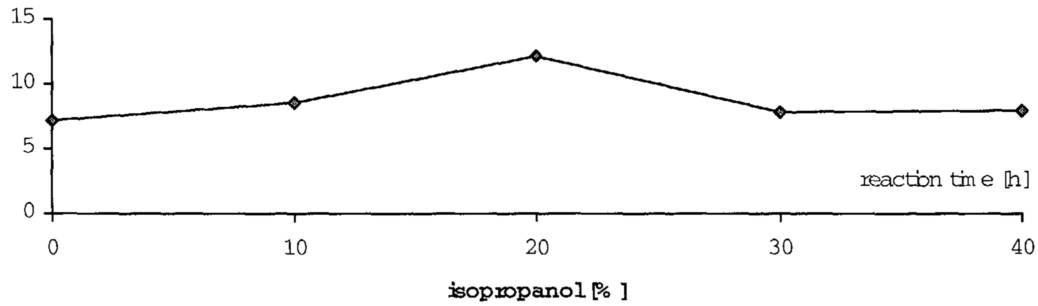


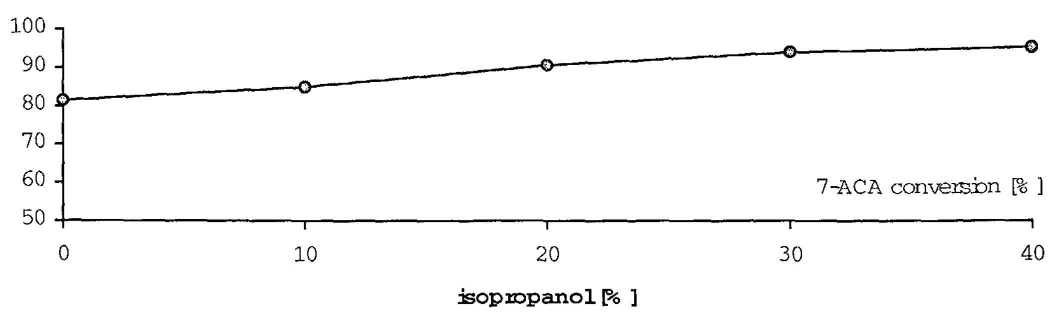


As expected 7-ACA conversion is faster and better at higher solvent content, since the dissociation constant for ThAH is shifted to alkaline.

**Example 2: influence of water-soluble solvents on the thermodynamically controlled synthesis of cephalotin at high educt concentration:**

The experiments were performed in 400 ml scale with 20 kU/l PGA-450, 150 mmol/l 7-ACA, 600 mmol/l ThAH,

2 mol/l HCl for adjusting the pH according to the first diagram, 20°C and 250 rpm:

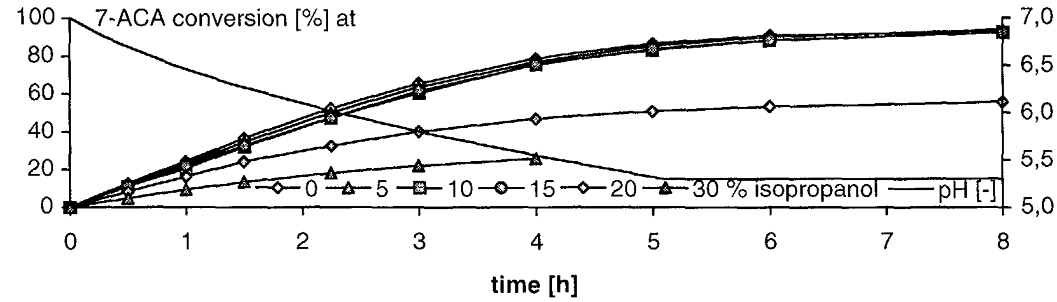


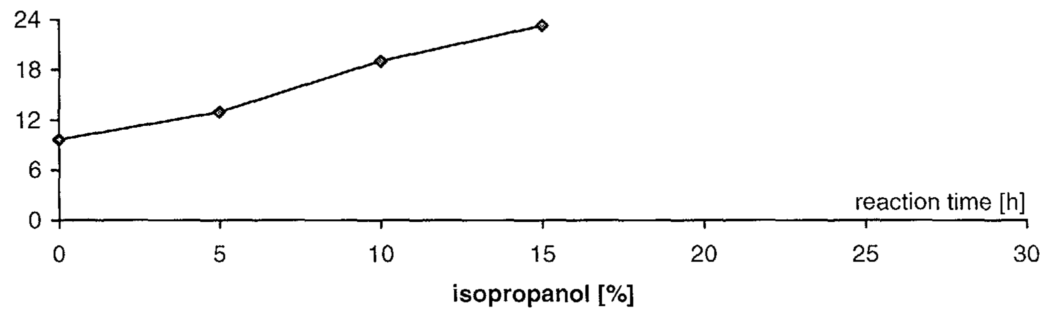


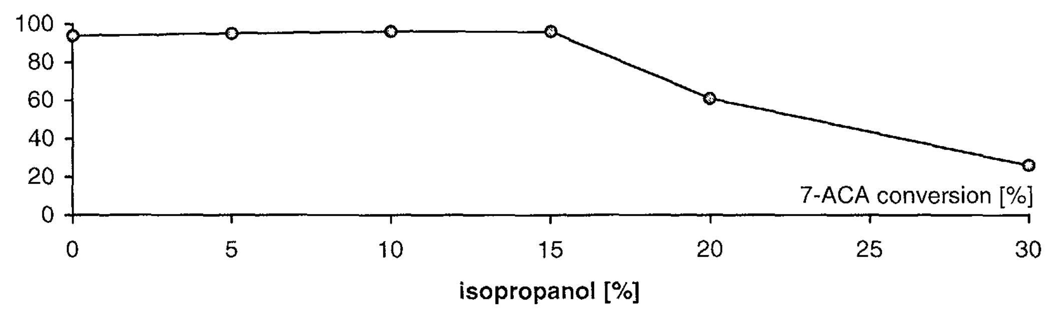


At the modified reaction conditions 3-fold more 7-ACA and ThAH are charged and a pH gradient is applied. Now the results are opposite to the previous trial. With increasing isopropanol concentration slightly better yields are obtained, but reaction time gets much longer. At too high isopropanol input 7-ACA conversion is too slow for avoiding 7-ACA precipitation at reduced pH causing a stop of further cephalotin synthesis.

**Example 3: ThAH recovery and cephalotin isolation:**

We surprisingly found, that for extraction only toluene or xylene are suitable for separating ThAH from cephalotin. In a typical protocol 50 % toluene or xylene {% are based on the initial volume} are added to the reaction solution. While stirring rapidly at room temperature pH 3.6 is adjusted e. g. by 25 % HCl. Although ThAH has a p$K_s$ value of 3.9 a lower pH is not feasibly as cephalotin might form an oily paste. Before separating layers filtrate the solution from tar impurities, which are introduced by typical ThAH quality.

At pH 3.6 several extractions have to be performed for removing ThAH from reaction solution completely. Alternatively the organic layer can be re-extracted with e. g. 25 % water at pH 6.0 while stirring rapidly. Repeat the extraction of the reaction solution with the recycled solvent at pH 3.6 followed by re-extraction with 20, 15, 10 and 5 % water at pH 6.0.

The ThAH containing re-extracts are combined and either precipitated at low pH or it is directly reused in next cycle.

The cephalotin in the reaction solution is either precipitated at low pH as free acid or preferably salted out by

known procedures [Pfeiffer R. R., Yang K. S. (Eli Lilly); Deutsche Offenlegungsschrift DE 2164399 (1970); Gewinnung von Cephalothinsalzen].

The recovery yield of thienylacetic acid was determined in four repetitive cycles. The experiments were performed in 400 ml scale with 10 kU/l PGA-450, 100 mmol/l 7-ACA, 400 mmol/l ThAH, 2 mol/l HCl for pH adjustment, 20°C and 250 rpm.

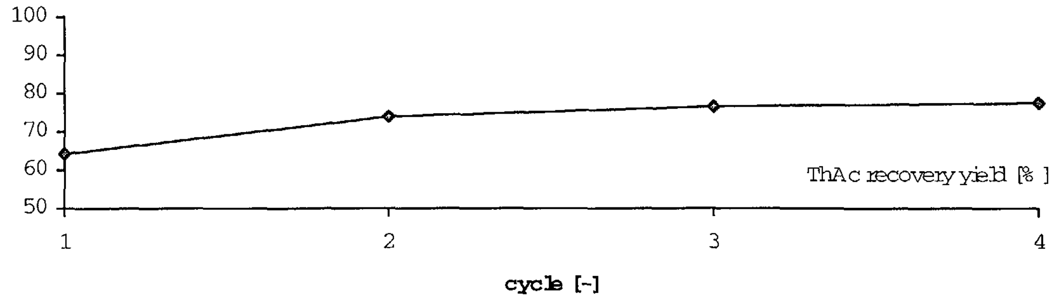


The recovery yield in the first cycles is lower, since fresh PGA-450 is used in the beginning. After three cycles an equilibrium is reached, where nearly all ThAH is either converted to cephalotin or isolated. Main impurity in recovered ThAH is cephalotin, which is not lost by reusing the recovered ThAH.

**Example 4: influence of water-soluble solvents on the thermodynamically controlled synthesis of MA-7-ACA at high educt concentration:**

The experiments were performed in 400 ml scale with 13 kU/l PGA-450, 150 mmol/l 7-ACA, 600 mmol/l D-mandelic acid, 2 mol/l HCl for adjusting the pH according to the first diagram, 20°C and 250 rpm:

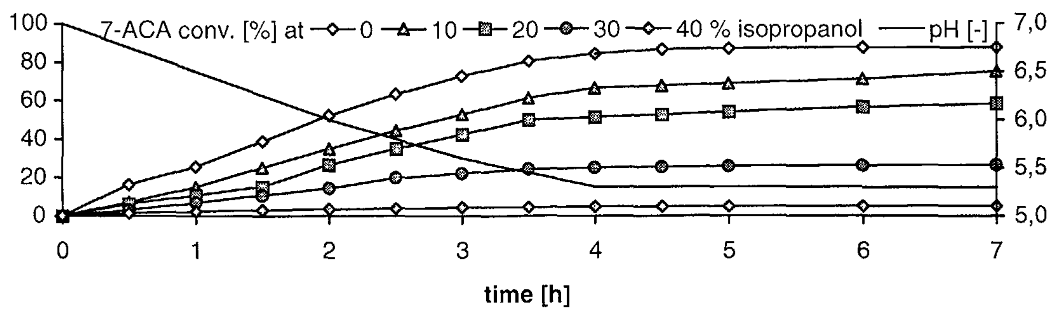


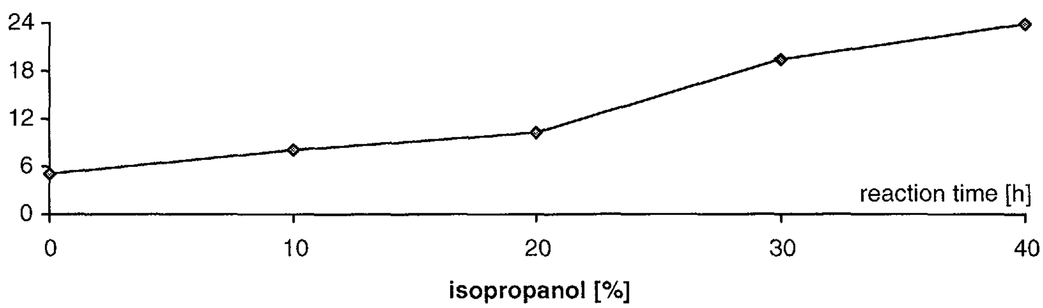

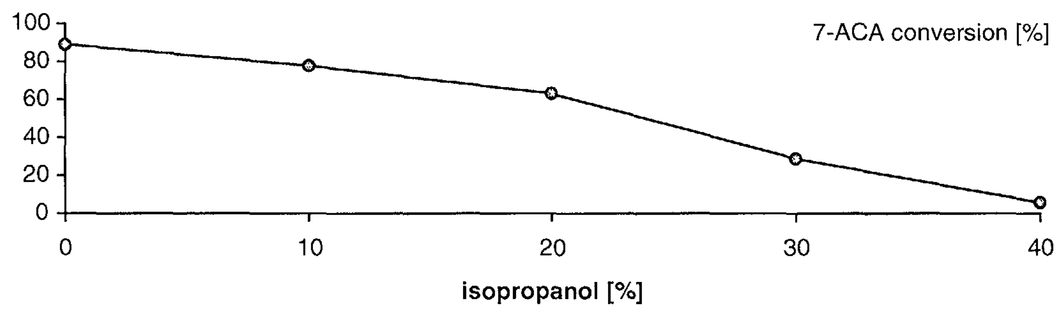


Reaction conditions are almost identical to example 2, but PGA-450 input was reduced to 13 kU/l. MA-7-ACA formation gets worse with increasing isopropanol input. As in example 2 for cephalotin (at same high substrate input) reaction time gets longer.

**Example 5: kinetically controlled synthesis of MA-7-ACA using the methyl or hydroxyethylester for activation:**

The experiment was performed in 400 ml scale with 2.5 kU/l PGA-450, 150 mmol/l 7-ACA, 300 mmol/l MAM, 10°C, pH 6.5 by 2 mol/l NaOH and 250 rpm:

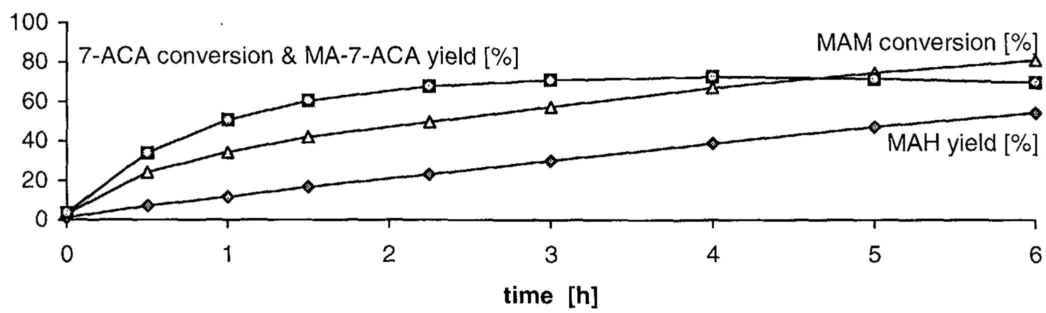


72.8 % maximum MA-7-ACA yield is obtained after 4 h. At higher side chain excess a little bit higher yields are achieved e. g. 79.0 % cefamandol with 400 mmol/l MAM.

The experiment was performed in 400 ml scale with 2.5 kU/l PGA-450, 150 mmol/l 7-ACA, 300 mmol/l MAG prepared from D-mandelic acid and 7-fold ethylene glycol at 40°C without further purification, pH 7.0 by 2 mol/l NaOH, 10°C and 250 rpm:

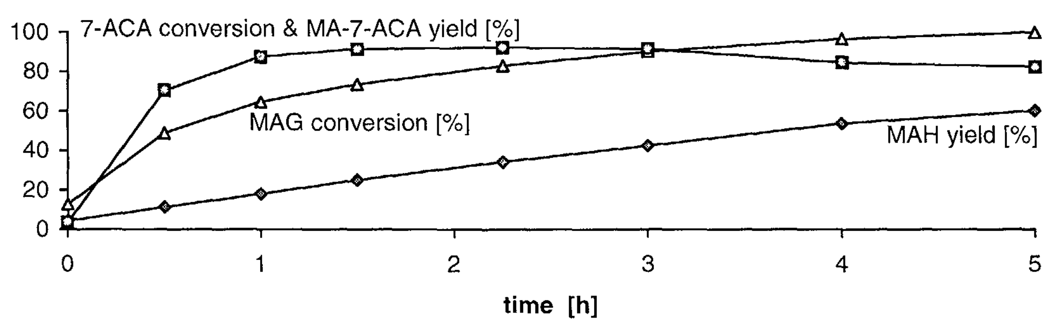


92.2 % maximum MA-7-ACA yield is obtained after 2.25 h. Conversion is much faster and higher as using the methylester for activation and even better as for the thermodynamically controlled synthesis with 90.6 % yield.

**Example 6: kinetically controlled synthesis of cefamandol using the methyl- or hydroxyethylester for activation:**

The experiment was performed in 400 ml scale with 2 kU/l PGA-450, 150 mmol/l TZA, 300 mmol/l MAM, 10°C, pH 6.5 by 2 mol/l NaOH and 250 rpm:

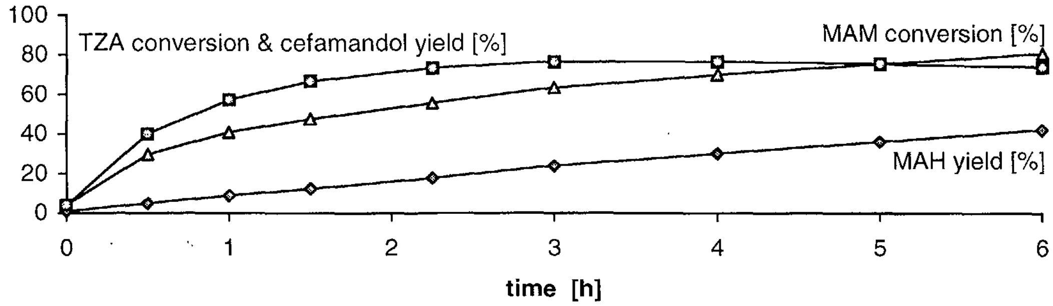


76.5 % maximum cefamandol yield is obtained after 4 h. At higher side chain excess a little bit higher yields are achieved e. g. 82.2 % cefamandol with 400 mmol/l MAM.

The experiment was performed in 400 ml scale with 2.5 kU/l PGA-450, 150 mmol/l TZA, 300 mmol/l MAG prepared from D-mandelic acid and 7-fold ethylene glycol at 40°C without further purification, pH 7.0 by 2 mol/l NaOH, 10°C and 250 rpm:

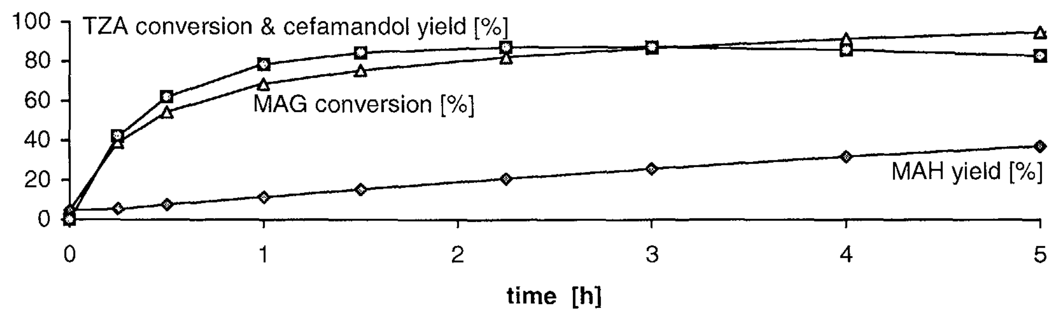


87.2 % maximum MA-7-ACA yield is obtained after 3 h. At higher side chain excess a little bit higher yields are achieved e. g. 90.8 % cefamandol with 400 mmol/l MAG. Conversion is much faster and higher as using the methylester for activation, but approximately 5 % lower as in the corresponding MA-7-ACA trials.

As alternative the thermodynamical approach is not feasible, as the window is too small, where D-mandelic acid is undissoziated and TZA is soluble.

**Example 7: influence of TDA concentration on kinetically controlled synthesis of cefazolin:**

The experiments were performed in 400 ml scale with 5 kU/l PGA-450, equimolar amounts of TDA and TzAM, but feeding after 0.5, 1.0, 1.5 and 2.0 h additional half equimolar amounts of TzAM resulting in a 3-fold ratio to TDA, pH 7.5 until 0.5 h, pH 7.35 until 1.0 h, pH 7.2 until 1.5 h, pH 7.1 until 2.0 h and afterwards pH 7.0 by 2 mol/l NaOH, 10°C and 250 rpm:

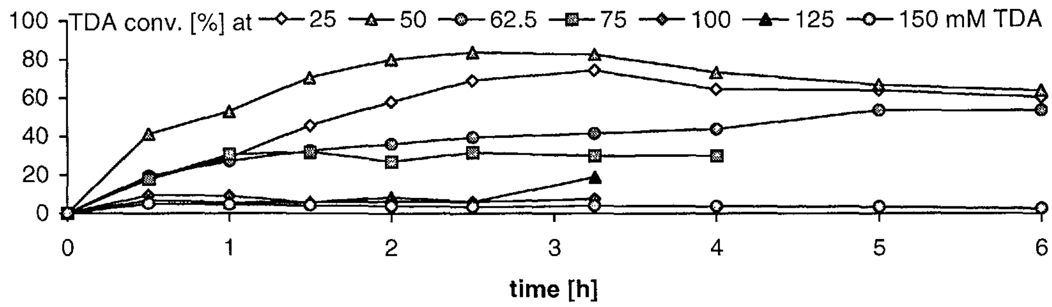


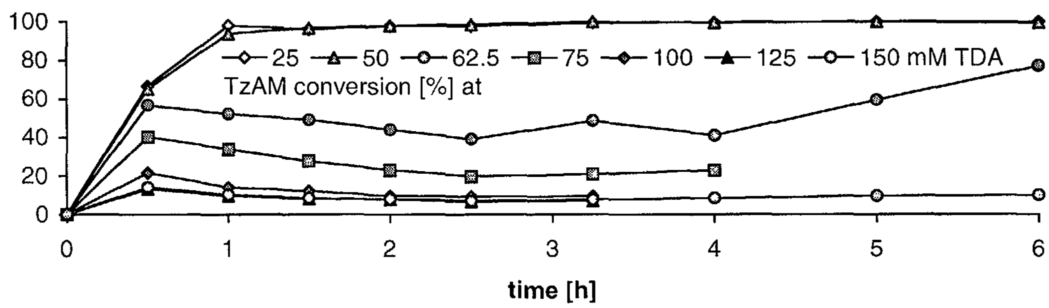


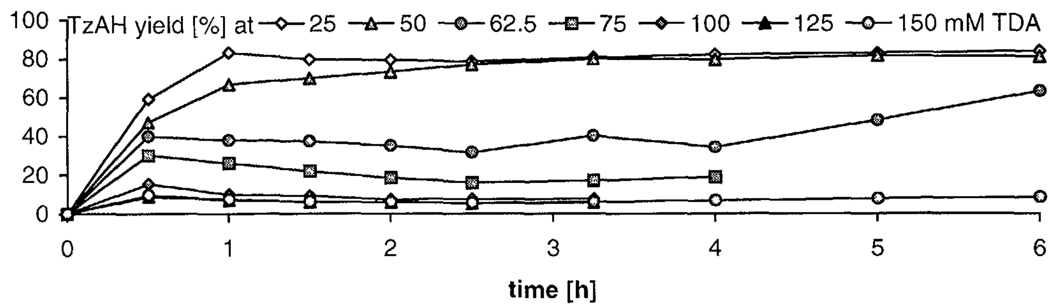


Kinetically controlled synthesis of cefazolin from TDA and TzAH preferably activated as methylester is hindered by the low solubility of TDA at reasonably pH. At higher pH, where TDA is soluble, TzAM is hydrolysed fast by PGA. We could surprisingly overcome this problem by charging preferably 50 - 60 mM TDA, applying a pH gradient and feeding solid TzAM during synthesis. 83.8 % maximum cefazolin yield is obtained after 2.5 h.

**Example 8: kinetically controlled synthesis of TzA-7-ACA and isolation:**

The experiments were performed in 1000 ml scale with 1.5 kU/l PGA-450, 150 mmol/l 7-ACA, 262.5 mmol/l TzAM, pH 7.0 by 2 mol/l NaOH, 10°C and 250 rpm:

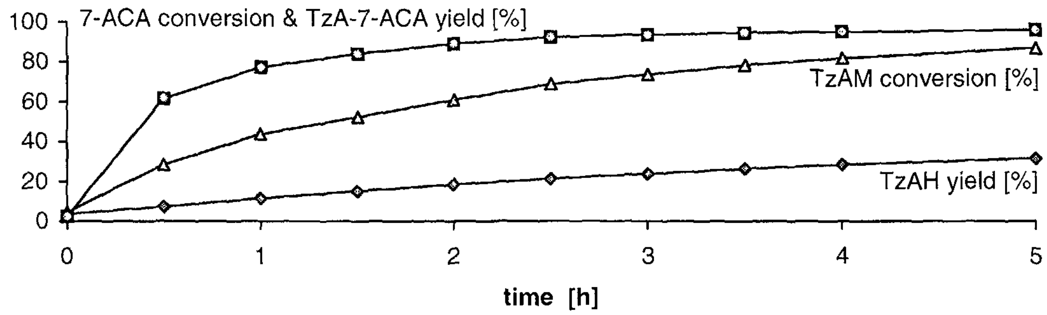


Surprisingly with only a 1.75 fold ratio of TzAM to 7-ACA 94.5 - 97.5 % yield are obtained. Same yield was reported before charging 3-fold the side chain (see background of invention), which is much less economic.

TzA-7-ACA can be salted out e. g. by sodium or potassium salts. For example 15 - 30 % preferably 20 % NaCl are added to the reaction solution. The salt precipitate is filtered off and washed with acetone. At preferred conditions 67.9 % TzA-7-ACA Na in 98.6 % HPLC purity is obtained. Less then 0.2 % 7-ACA are detectable as impurity.

**Example 9: 3-processing of TzA-7-ACA with MMTD to cefazolin:**

150 mmol/l isolated TzA-7-ACA Na with less than 0.1 % 7-ACA impurity and 150 mmol/l MMTD are dissolved at pH 6.5 and adjusted 5 h to 65°C until the cefazolin maximum is reached:

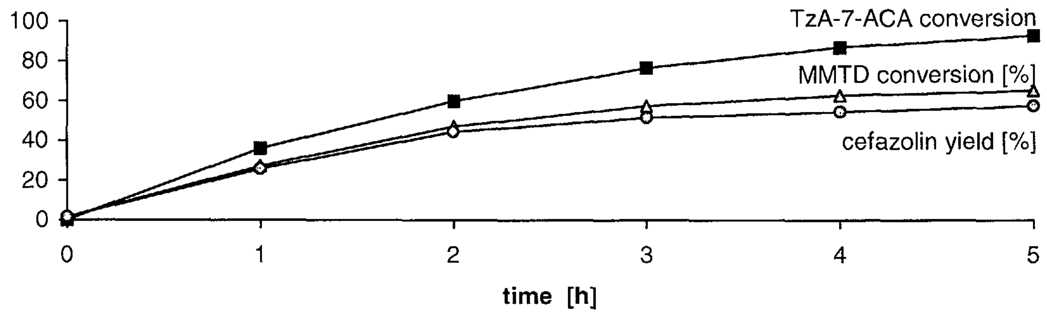


The solution turned from light yellow to yellow as indicated by following scan:

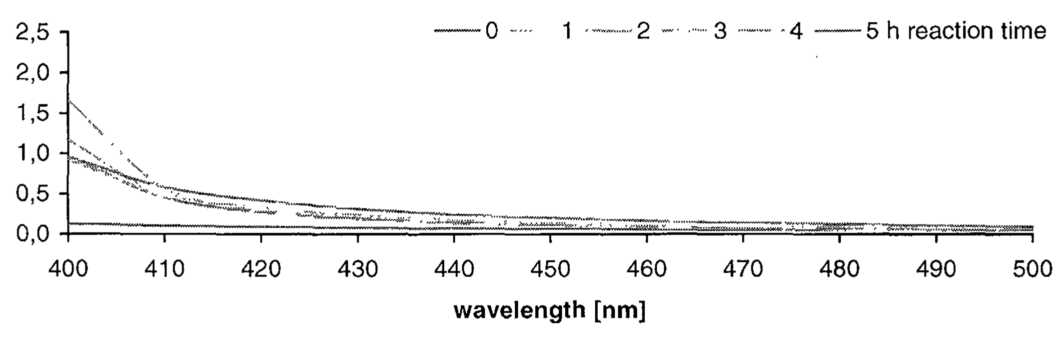


The MMTD in the reaction solution is extracted by n-butylacetate. The cefazolin in the aqueous layer is precipitated at pH 1.5, filtrated and washed with acetone. 59.6 % cefazolin in white colour are obtained corresponding to 41.2 % total yield from 7-ACA. Based on HPLC analysis the product contains 97.9 % cefazolin, 0.2 % MMTD and 1.9 % TzA-7-ACA.

According to example 10 TzA-7-ACA reaction solution is produced in 94.5 % yield. MMTD equimolar to the 7-ACA input is dissolved at pH 6.5. The solution is adjusted 5 h to 65°C until the cefazolin maximum is reached. The

solution turned from yellow to light brown as indicated by following scan:

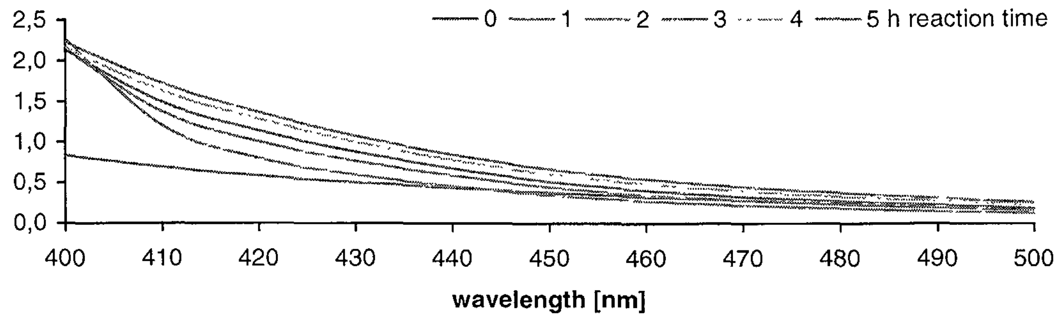


The formed cefazolin is isolated as disclosed before, but washed more intensively with acetone in order to remove colour. Nevertheless a light beige product in 41.1 % yield is obtained. Based on HPLC analysis the product contains 96.0 % cefazolin, 0.3 % MMTD, 1.8 % TzA-7-ACA and 1.9 % TDA (by 3-processing of unconverted 7-ACA).

According to these data, a much better product quality without TDA pollution is obtained by salting out the TzA-7-ACA before 3-processing it to cefazolin without higher losses in yield.

**Example 10: thermodynamically controlled synthesis of cefoxitin:**

We describe the first time the thermodynamically controlled synthesis of cefoxitin from 7-MACA and ThAH as free acid <it will be possible>.

**Example 11: kinetically controlled synthesis of cefoxitin:**

We describe the first time the kinetically controlled synthesis of cefoxitin from 7-MACA and ThAH activated e. g. as methylester <it will be possible>.

**Example 12: thermodynamically controlled synthesis of cefazedon:**

We describe the first time the thermodynamically controlled synthesis of cefazedon from TZA and 3,5-dichlor-4-pyridon-1-yl-acetic acid as free acid <to be evalutated>.

**Example 13: kinetically controlled synthesis of cefazedon:**

We describe the first time the kinetically controlled synthesis of cefazedon from TZA and 3,5-dichlor-4-pyridon-1-yl-acetic acid activated as methyl- or hydroxyethylester <to be evaluated>.

**Example 14: kinetically controlled synthesis of ceftexol:**

We describe the first time the kinetically controlled synthesis of ceftexol from TA with TzAH preferably activated as methylester <it will be possible>.

**Example 15: kinetically controlled synthesis of p-hydroxyphenylglycyl-7-ACA using the methyl or hydroxyethylester for activation:**

The experiment was performed in 400 ml scale with 5 kU/l PGA-450, 150 mmol/l 7-ACA, 300 mmol/l HPM, 10°C, pH 7.0 by 2 mol/l NaOH and 250 rpm. As no standard was available, it was assumed that the molar extinction coefficient of HP-7-ACA is the sum of the molar extinction coefficients of HPH and 7-ACA:

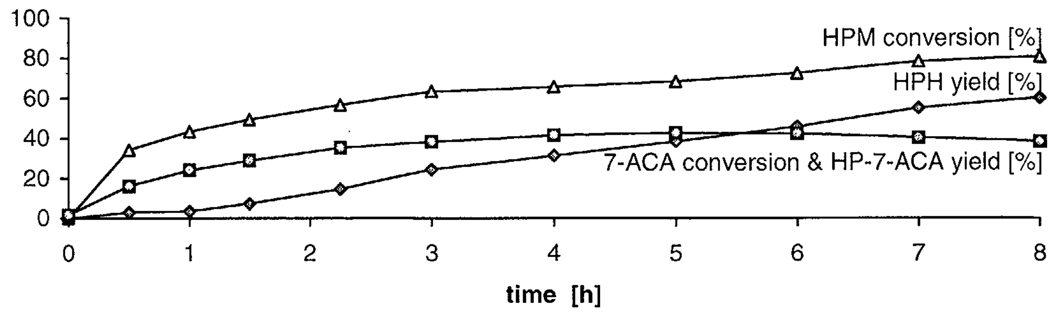


We describe the first time the kinetically controlled synthesis of p-hydroxyphenylglycyl-7-ACA from 7-ACA and HPM. 42.7 % maximum HP-7-ACA yield is obtained after 5 h.

The experiment was performed in 400 ml scale with 2 kU/l PGA-450, 150 mmol/l 7-ACA, 300 mmol/l HPG prepared from HPH and 5-fold ethylene glycol at 55°C without further purification, pH 6.5 by 2 mol/l NaOH, 10°C and 250 rpm:

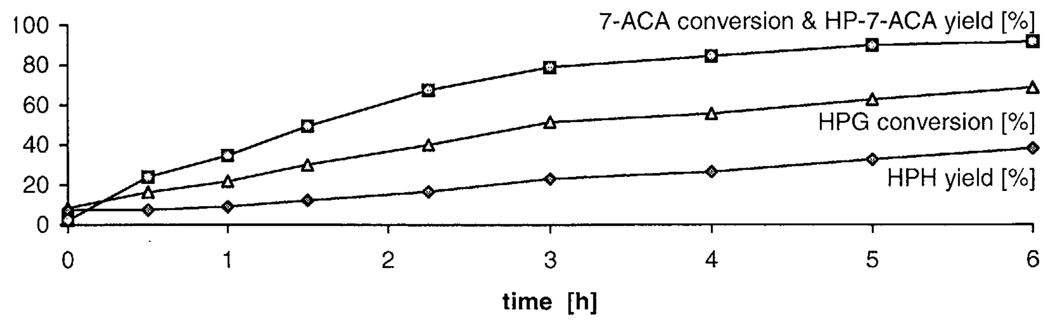


With 40 % biocatalyst input 91.5 % maximum HP-7-ACA yield is obtained after 6 h. Conversion is much faster and HP-7-ACA yield more than double so high as using the methylester for activation.

**Example 16: kinetically controlled synthesis of p-hydroxyphenylglycyl-TZA:**

The experiment was performed in 400 ml scale with 2 kU/l PGA-450, 150 mmol/l TZA, 300 mmol/l HPG prepared from HPH and 5-fold ethylene glycol at 55°C without further purification, pH 7 by 2 mol/l NaOH, 10°C and 250 rpm. As no standard was available, it was assumed that the molar extinction coefficient of HP-TZA is the sum of the molar extinction coefficients of HPH and TZA:

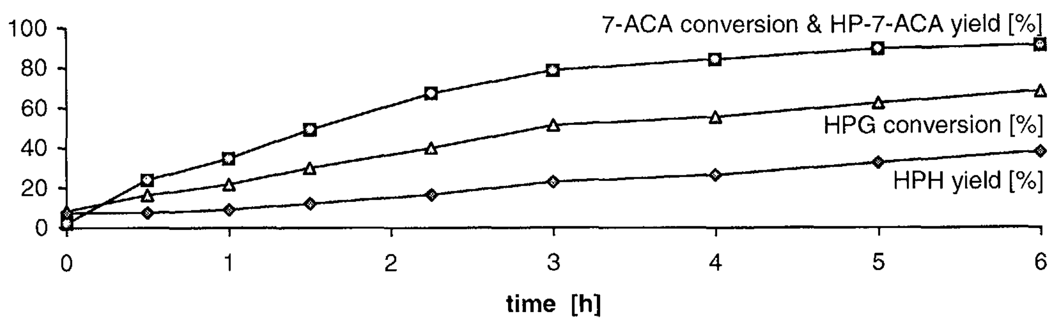


We describe the first time the kinetically controlled synthesis of p-hydroxyphenylglycyl-TZA from TZA and HPH preferably activated as hydroxyethylester. 86.0 % maximum HP-7-ACA yield is obtained after 4.5 h.

**Example 17: kinetically controlled synthesis of cefatrizin:**

We describe the first time the kinetically controlled synthesis of cefatrizin from MTA and HPH preferably activated as hydroxyethylester <it will be possible>.

**Example 18: thermodynamically controlled synthesis of n-formylamino-4-thiazole acetyl-7-ACA:**

We describe the first time the thermodynamically controlled synthesis of n-formylamino-4-thiazole acetyl-7-ACA from 7-ACA and 2-formylamino-4-thiazole acetic acid as free acid <to be evaluated>.

**Example 19: kinetically controlled synthesis of n-formylamino-4-thiazole acetyl-7-ACA:**

We describe the first time the kinetically controlled synthesis of n-formylamino-4-thiazole acetyl-7-ACA from 7-ACA and 2-formylamino-4-thiazole acetic acid activated e. g. as ethylester <to be evaluated>.

**Example 20: thermodynamically controlled synthesis of n-formyl-cefotiam:**

We describe the first time the thermodynamically controlled synthesis of n-formyl-cefotiam from DZA and 2-formylamino-4-thiazole acetic acid as free acid <to be evaluated>.

**Example 21: kinetically controlled synthesis of n-formyl-cefotiam:**

We describe the first time the kinetically controlled synthesis of n-formyl-cefotiam from DZA and 2-formylamino-4-thiazole acetic acid activated e. g. as ethylester <to be evaluated>.

**Example 22: thermodynamically controlled synthesis of cefapirin:**

We describe the first time the thermodynamically controlled synthesis of cefapirin from 7-ACA and 4-pyridylthioacetic acid as free acid <to be evaluated>.

**Example 23: kinetically controlled synthesis of cefapirin:**

We describe the first time the kinetically controlled synthesis of cefapirin from 7-ACA and 4-pyridylthioacetic acid activated as methyl- or hydroxyethylester <to be evaluated>.

**Example 24: thermodynamically controlled synthesis of cefacetril:**

We describe the first time the thermodynamically controlled synthesis of cefacetril from 7-ACA and cyanoacetic acid as free acid <it will be possible>.

**Example 25: kinetically controlled synthesis of cefacetril:**

The experiment was performed in 400 ml scale with 5 kU/l PGA-450, 150 mmol/l 7-ACA, 300 mmol/l CNM, pH 7 by 2 mol/l NaOH, 10°C and 250 rpm. As no standard was available, it was assumed that the molar extinction coefficient of cefacetril is equal to 7-ACA. Cyanoacetic acid and its methylester cannot be determined by HPLC due to a missing chromophore:

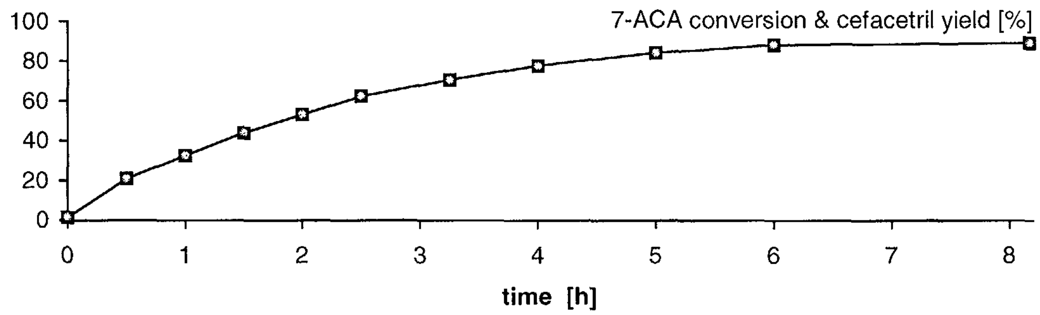


We describe the first time the synthesis of cefacetril from 7-ACA and cyanoacetic acid activated e. g. as methylester. 89.3 % maximum cefacetril yield is obtained after 8.2 h.

**Example 26: enzymatic cleavage of cephamycin C to 3-aminocarbonyloxymethyl-7-methoxy-3-cephem-4-carbonic acid:**

We describe the first time the enzymatic cleavage of cephamycin C to 3-aminocarbonyloxymethyl-7-methoxy-3-cephem-4-carbonic acid (7-MACA), DAO and GAC <to be evaluated>.

## Claims

**1.** An enzymatic process for coupling the 7-amino group of a cephalosporin nucleus with the carboxylic function of an acetic acid derivative consisting of a:

- residue with π-electrons (phenyl-, pyridyl-, thienyl-, tetrazolyl-, CN- etc.)
- short spacer ($-CH_2-$, -CHOH-, $CHNH_2-$, $-OCH_2-$, $-SCH_2-$ etc.)
- carboxylic function or derivative (-COOH, COOR, CONHR),

which is **characterised by**:

➢ using a penicillin amidase (EC 3.5.1.11) or α-amino acid esterase (EC 3.1.1.43) from Acetobacter, Achromobacter, Aerobacter, Aerococcus, *Aeromonas, Alcaligenes, Aphanocladium, Arthrobacter, Azotobacter, Bacillus, Beneckea, Brevibacterium, Cellulomas, Cephalosporium, Clostridium, Comamonas, Corynebacterium, Erwinia, Escherichia coli, Flavobacterium, Gluconobacter suboxidans, Klebsiella aerogenes, Kluyvera citrophlia, Microbacterium lacticum, Micrococcus, Mycoplana, Norcardia, Paracoccus, Paracolon bacilli, Piocianus, Protaminobacter, Proteus, Pseudobacterium, Pseudomonas, Rhodococus rhodochrous, Rhodopseudomonas, Sacina, Samonella, Serratia, Shigella, Soil bacterium, Spirillum, Staphylococcus, Streptomyces, Thiobacillus,* Xantomonas or Yersinia species as free enzyme or in any suitable immobilised form,
➢ applying no or maximum 10 % organic solvent,
➢ applying at least 100 mmol/l cephalosporin nucleus as long as it is soluble, otherwise 50 - 100 % of its maximum solubility is charged,
➢ applying the free organic acid in a 3 - 5 fold molar ratio or activated as ester or amide in a 1 - 3 fold molar ratio,
➢ adjusting pH 5.0 - 8.0 and 0 - 40°C.

**2.** An enzymatic process under thermodynamical control according claim 1, where:

➢ no or maximum 10 % organic solvent is used,
➢ at least 100 mmol/l cephalosporin nucleus is applied,
➢ the free organic acid is charged in a 3 - 5 fold molar ratio,
➢ the pH is reduced by acid according the reaction progress from initially pH 7.5 - 6.25 to pH 6.25 - 5.0,
➢ 10 - 40°C is adjusted.

**3.** An enzymatic process according claim 2, where the organic acid is ThAH, the cephalosporin nucleus is 7-ACA or 7-MACA and the formed product is cephalotin or cefoxitin.

**4.** An enzymatic process according claim 2, where the organic acid is D-mandelic acid, the cephalosporin nucleus is 7-ACA and the formed product is mandoyl-7-ACA.

**5.** An enzymatic process according claim 2, where the organic acid is 3,5-dichlor-4-pyridon-1-yl-acetic acid, the cephalosporin nucleus is 7-ACA or TZA and the formed product is 3,5-dichlor-4-pyridon-1-yl-acetyl-7-ACA or cefazedon.

**6.** An enzymatic process according claim 2, where the organic acid is 2-formylamino-4-thiazole acetic acid, the cephalosporin nucleus is 7-ACA or DZA and the formed product is n-formylamino-4-thiazole acetyl-7-ACA or n-formylcefotiam.

**7.** An enzymatic process according claim 2, where the organic acid is 4-pyridylthioacetic acid, the cephalosporin nucleus is 7-ACA and the formed product is cefapirin.

**8.** An enzymatic process according claim 2, where the organic acid is cyanoacetic acid, the cephalosporin nucleus is 7-ACA and the formed product is cefacetril.

**9.** An enzymatic process under kinetical control according claim 1, where:

- ➢ no organic solvent is used,
- ➢ at least 100 mmol/l cephalosporin nucleus is applied as long as it is soluble, otherwise 50 - 100 % of its maximum solubility is charged,
- ➢ the organic acid is charged as ester or amide in a 1 - 3 fold molar ratio,
- ➢ pH 6.0 - 8.0 is titrated by alkaline,
- ➢ 0 - 30°C are adjusted.

**10.** An enzymatic process according claim 9, where the organic acid is activated as methyl- or ethylester. It is charged in one portion or initially equimolar to the cephalosporin nucleus with further feed during synthesis.

**11.** An enzymatic process according claim 9, where the organic acid is activated as hydroxyethylester, prepared from 3 - 10 fold ethylene glycol and might be used without further purification.

**12.** An enzymatic process according claims 9 and 10, where the organic acid is ThAH, the cephalosporin nucleus is 7-ACA or 7-MACA and the formed product is cephalotin or cefoxitin.

**13.** An enzymatic process according claims 9 and 11, where the organic acid is D-mandelic acid, the cephalosporin nucleus is 7-ACA or TZA and the formed product is mandoyl-7-ACA or cefamandol.

**14.** An enzymatic process according claims 9 - 11, where the organic acid is 3,5-dichlor-4-pyridon-1-yl-acetic acid, the cephalosporin nucleus is 7-ACA or TZA and the formed product is 3,5-dichlor-4-pyridon-1-yl-acetyl-7-ACA or cefazedon.

**15.** An enzymatic process according claims 9 - 11, where the organic acid is 2-formylamino-4-thiazole acetic acid, the cephalosporin nucleus is 7-ACA or DZA and the formed product is n-formylamino-4-thiazole acetyl-7-ACA or n-formyl-cefotiam.

**16.** An enzymatic process according claims 9 - 11, where the organic acid is 4-pyridylthioacetic acid, the cephalosporin nucleus is 7-ACA and the formed product is cefapirin.

**17.** An enzymatic process according claims 9 and 10, where the organic acid is cyanoacetic acid, the cephalosporin nucleus is 7-ACA and the formed product is cefacetril.

**18.** An enzymatic process according claims 9 and 11, where the organic acid is p-hydroxyphenylglycine, the cephalosporin nucleus is 7-ACA, TZA or MTA and the formed product is p-hydroxyphenylglycyl-7-ACA, p-hydroxyphenylglycyl-TZA or cefatrizin.

**19.** An enzymatic process according claims 9 and 10, where the organic acid is TzAH, the cephalosporin nucleus is 7-ACA, TDA or TA and the formed product is TzA-7-ACA, cefazolin or ceftexol.

**20.** An enzymatic process according claim 19, where 50 - 60 mmol/l TDA is charged.

**21.** An enzymatic process according claim 20, where TzAM is charged equimolar to TDA with further feed during synthesis.

**22.** An enzymatic process according claims 20 and 21, where the pH is reduced by acid according the reaction progress from initially pH 7.2 - 7.5 to pH 6.3 - 7.0.

**23.** A method for downstreaming, where TzA-7-ACA produced according claim 19 is salted out by sodium or potassium salts.

**24.** A method for downstreaming according claim 23, where TzA-7-ACA produced according claim 19 is salted out by 15 - 30 % NaCl.

**25.** A process for 3-processing of a enzymatically synthesised 7-derivative of 7-ACA with a heterocyclic thiol in aqueous solution, where:

- the 7-derivative of 7-ACA is polluted with less than 0.2 % 7-ACA,
- 50 - 250 mmol/l 7-derivative of 7-ACA are charged,
- the heterocyclic thiol is charged at least equimolar to the cephalosporin,
- pH 6 - 8.5 is adjusted,
- 50 - 75°C are applied for 3-processing

**26.** A process according claim 25, where the heterocylic thiol is MMTD, the 7-derivative of 7-ACA is TzA-7-ACA and the formed product is cefazolin.

**27.** A process according claim 26, where the used TzA-7-ACA is salted out according claims 23 or 24.

**28.** A method for downstreaming, where ThAH is removed from an aqueous cephalotin solution by extraction with toluene or xylene at pH 3.5 - 3.9.

**29.** An process for enzymatic cleavage of cephamycin C to 7-MACA, where

- 50 - 150 mmol/l cephamycin C is oxidatively deaminated in presence of oxygen at pH 7.0 - 8.0 and 10 - 30°C by DAO,
- the formed ketointermediate is spontaneously decarboxylated by the by-product hydrogen peroxide,
- for completing the decarboxylation external $H_2O_2$ might be charged, which is decomposed afterwards by catalase or metal catalyst,
- the formed glutaryl-7-MACA is hydrolysed to 7-MACA at pH 7.5 - 8.5 and 10 - 30°C by GAC.

European Patent Office

# EUROPEAN SEARCH REPORT

Application Number
EP 02 07 8608

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | FERNANDEZ-LAFUENTE R ET AL.: "Dynamic reaction design of enzymic biotransformations in organic media: equilibrium - controlled synthesis of antibiotics by penicillin G acylase." BIOTECHNOLOGY AND APPLIED BIOCHEMISTRY, (1996 OCT) 24 ( PT 2) 139-43., vol. 24, no. 2, October 1996 (1996-10), pages 139-143, XP000989462 * the whole document * --- | 1-8 | C12P35/04 C12P35/00 C12P35/02 |
| Y | WO 99 20786 A (DSM NV ;DOOREN THEODORUS JOHANNES GODF (NL); MOODY HAROLD MONRO (N) 29 April 1999 (1999-04-29) * the whole document * * page 10, line 17 - page 11, line 3 * * page 12, line 6-32 * * claims 1-16 * --- | 1-8 | |
| A | SCHROËN C G P H ET AL.: "Thermodynamically controlled synthesis of beta-lactam antibiotics. Equilibrium concentrations and side-chain properties" ENZYME AND MICROBIAL TECHNOLOGY, vol. 24, no. 8-9, June 1999 (1999-06), pages 498-506, XP002233337 * the whole document * --- | 1,2 | TECHNICAL FIELDS SEARCHED (Int.Cl.7) C12P |
| A | FERNANDEZ-LAFUENTE R ET AL.: "Synthesis of antibiotics (cephaloglycin) catalyzed by penicillin G acylase: Evaluation and optimization of different synthetic approaches" ENZYME AND MICROBIAL TECHNOLOGY, vol. 19, no. 1, July 1996 (1996-07), pages 9-14, XP001098845 * the whole document * --- -/-- | 1,2 | |

~~The present search report has been drawn up for all claims~~

| Place of search | Date of completion of the search | Examiner |
|---|---|---|
| THE HAGUE | 10 June 2003 | van de Kamp, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 02 07 8608

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int.Cl.7) |
| D,A | EP 0 458 932 A (CONSEJO SUPERIOR INVESTIGACION) 4 December 1991 (1991-12-04) * the whole document * * example 8 * --- | 1-3 | |
| D,A | WO 98 04732 A (SQUIBB BRISTOL MYERS CO) 5 February 1998 (1998-02-05) * examples 1-3 * * claims 1-6 * --- | | |
| A | SHAW SHYH-YU ET AL.: "Enzymatic synthesis of cephalothin by penicillin G acylase." ENZYME AND MICROBIAL TECHNOLOGY, vol. 26, no. 2-4, February 2000 (2000-02), pages 142-151, XP002233338 ISSN: 0141-0229 * the whole document * --- | | |
| D,A | JUSTIZ O H ET AL.: "One-pot chemoenzymatic synthesis of 3'-functionalized cephalosporines ( cefazolin ) by three consecutive biotransformations in fully aqueous medium" JOURNAL OF ORGANIC CHEMISTRY, vol. 62, no. 26, 26 December 1997 (1997-12-26), pages 9099-9106, XP002233339 * the whole document * --- | | TECHNICAL FIELDS SEARCHED (Int.Cl.7) |
| A | KARADY S ET AL.: "The chemistry of cephamycins VI. Cleavage of the 7-amido group" TETRAHEDRON LETTERS, no. 5, 1978, pages 407-408, XP002233340 ISSN: 0040-4039 * the whole document * --- -/-- | | |

~~The present search report has been drawn up for all claims~~

| Place of search | Date of completion of the search | Examiner |
|---|---|---|
| THE HAGUE | 10 June 2003 | van de Kamp, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 02 07 8608

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,A | MUNEYUKI R ET AL.: "Water-soluble esters: Useful enzyme substrates for the synthesis of beta-lactam antibiotics." CHEMISTRY AND INDUSTRY, vol. 5, 1981, pages 159-161, XP008014281 * the whole document * --- | | |
| A | BRUGGINK A ET AL.: "Penicillin acylase in the industrial production of beta-lactam antibiotics" ORGANIC PROCESS RESEARCH & DEVELOPMENT, vol. 2, no. 2, March 1998 (1998-03), pages 128-133, XP002233341 * abstract * --- | | |
| D,A | FERNANDEZ-LAFUENTE R ET AL.: "Equilibrium controlled synthesis of cephalothin in water-cosolvent systems by stabilized penicillin G acylase." APPL. BIOCHEM. BIOTECHNOL., vol. 27, no. 3, 1991, pages 277-290, XP008014229 * abstract * * page 287, line 32 - page 288, line 14 * --- | | TECHNICAL FIELDS SEARCHED (Int.Cl.7) |
| A | NIERSTRASZ V A ET AL.: "Thermodynamically controlled synthesis of cefamandole." BIOCATALYSIS AND BIOTRANSFORMATION, vol. 17, no. 3, 1999, pages 209-223, XP008014226 * the whole document * --- -/-- | | |

~~The present search report has been drawn up for all claims~~

| Place of search | Date of completion of the search | Examiner |
|---|---|---|
| THE HAGUE | 10 June 2003 | van de Kamp, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 02 07 8608

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | KASCHE V: "Mechanism and yields in enzyme-catalyzed equilibrium and kinetically controlled synthesis of beta-lactam antibiotics, peptides and other condensation products" ENZYME AND MICROBIAL TECHNOLOGY, vol. 8, no. 1, 1986, pages 4-16, XP008014254 ISSN: 0141-0229 * the whole document * ----- | | |
| | | | TECHNICAL FIELDS SEARCHED (Int.Cl.7) |

~~The present search report has been drawn up for all claims~~

| Place of search | Date of completion of the search | Examiner |
|---|---|---|
| THE HAGUE | 10 June 2003 | van de Kamp, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

Application Number

EP 02 07 8608

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1 (partially); 2-8 (all completely)

European Patent Office

## LACK OF UNITY OF INVENTION SHEET B

Application Number
EP 02 07 8608

The Search Division considers that the present European patent application does not comply with the requirements of unity of invention and relates to several inventions or groups of inventions, namely:

1. Claims: 1 (partially); 2-8 (all completely)

   An enzymatic process under thermodynamical control for coupling the 7-amino group of a cephalosporin nucleus with the carboxylic function of an acetic acid derivative using a penicillin amidase or alpha-amino acid esterase, applying no or maximum 10% organic solvent, at least 100 mM cephalosporin nucleus, the free organic acid is charged in a 3-5 fold molar ratio, the pH is reduced by acid according the reaction progress from initially pH 7.5-6.25 to 6.25-5.0, 10-40 C is adjusted, the formed product being cephalotin, cefoxitin, mandoyl-7-ACA, 3,5-dichlor-4-pyridon-1-yl-acetyl-7-ACA, cefazedon, n-formylamino-4-thiazole acetyl-7-ACA, n-formyl-cefotiam, cefapirin, and cefacetril.

2. Claims: 1,9,10,12 (all partially)

   An enzymatic process under kinetical control for coupling the 7-amino group of a cephalosporin nucleus with the carboxylic function of an acetic acid derivative using a penicillin amidase or alpha-amino acid esterase, applying no organic solvent, at least 100 mM cephalosporin nucleus as long as its soluble, otherwise 50-100% of its maximal solubility, the organic acid amide or methyl- or ethylester in a 1-3 fold molar ratio charged in one portion or initially equimolar to the cephalosporin nucleus with further feed during synthesis, the pH 6.0-8.0 is titrated by alkaline, 0-30 C is adjusted, the formed product being cephalotin.

3. Claims: 1,9,10,12 (all partially)

   An enzymatic process according to invention 2, the formed product being cefoxitin.

4. Claims: 1,9,11,13 (all partially)

   An enzymatic process under kinetical control for coupling the 7-amino group of a cephalosporin nucleus with the carboxylic function of an acetic acid derivative using a penicillin amidase or alpha-amino acid esterase, applying no organic solvent, at least 100 mM cephalosporin nucleus as long as its soluble, otherwise 50-100% of its maximal solubility, the organic acid amide or hydroxyethylester (prepared from 3-10 fold ethylene glycol) in a 1-3 fold molar ratio charged in one portion or initially equimolar to the cephalosporin nucleus with further feed during

European Patent Office

LACK OF UNITY OF INVENTION
SHEET B

Application Number
EP 02 07 8608

The Search Division considers that the present European patent application does not comply with the requirements of unity of invention and relates to several inventions or groups of inventions, namely:

synthesis, the pH 6.0-8.0 is titrated by alkaline, 0-30 C is adjusted, the formed product being mandoyl-7-ACA.

5. Claims: 1,9,11,13 (all partially)

An enzymatic process according to invention 4, the formed product being cefamandol.

6. Claims: 1,9-11,14 (all partially)

An enzymatic process under kinetical control for coupling the 7-amino group of a cephalosporin nucleus with the carboxylic function of an acetic acid derivative using a penicillin amidase or alpha-amino acid esterase, applying no organic solvent, at least 100 mM cephalosporin nucleus as long as its soluble, otherwise 50-100% of its maximal solubility, the organic acid amide or methyl- or ethylester or hydroxyethylester (prepared from 3-10 fold ethylene glycol) in a 1-3 fold molar ratio charged in one portion or initially equimolar to the cephalosporin nucleus with further feed during synthesis, the pH 6.0-8.0 is titrated by alkaline, 0-30 C is adjusted, the formed product being 3,5-dichlor-4-pyridon-1-yl-acetyl-7-ACA.

7. Claims: 1,9-11,14 (all partially)

An enzymatic process according to invention 6, the formed product being cefazedon.

8. Claims: 1,9-11,15 (all partially)

An enzymatic process according to invention 6, the formed product being n-formylamino-4-thiazole acetyl-7-ACA.

9. Claims: 1,9-11,15 (all partially)

An enzymatic process according to invention 6, the formed product being n-formyl-cefotiam.

10. Claims: 1,9-11 (all partially); 16 (completely)

An enzymatic process according to invention 6, the formed product being cefapirin.

European Patent Office

**LACK OF UNITY OF INVENTION SHEET B**

Application Number
EP 02 07 8608

The Search Division considers that the present European patent application does not comply with the requirements of unity of invention and relates to several inventions or groups of inventions, namely:

11. Claims: 1,9,10 (all partially); 17 (completely)

    An enzymatic process according to invention 2, the formed product being cefacetril.

12. Claims: 1,9,11,18 (all partially)

    An enzymatic process according to invention 4, the formed product being p-hydroxyphenylglycyl-7-ACA.

13. Claims: 1,9,11,18 (all partially)

    An enzymatic process according to invention 4, the formed product being p-hydroxyphenylglycyl-TZA

14. Claims: 1,9,11,18 (all partially)

    An enzymatic process according to invention 4, the formed product being cefatrizin.

15. Claims: 1,9,10,19 (all partially)

    An enzymatic process according to invention 2, the formed product being TzA-7-ACA.

16. Claims: 1,9,10,19 (all partially); 20-22 (completely)

    An enzymatic process according to invention 2, the formed product being cefazolin, with 50-60 mM TDA being charged, with TzAM being charged equimolar to TDA with further feed during synthesis, where the pH is reduced by acid according the reaction progress from initially pH 7.2-7.5 to pH 6.3-7.0.

17. Claims: 1,9,10,19 (all partially)

    An enzymatic process according to invention 2, the formed product being ceftexol.

18. Claims: 23,24 (both completely); 27 (partially)

    A method for downstreaming where TzA-7-ACA is salted out by sodium or potassium salts, e.g., by 15-30% NaCl.

European Patent Office

## LACK OF UNITY OF INVENTION SHEET B

Application Number
EP 02 07 8608

The Search Division considers that the present European patent application does not comply with the requirements of unity of invention and relates to several inventions or groups of inventions, namely:

19. Claims: 25,26 (both completely); 27 (partially)

    A process for 3-processing of an enzymatically synthesized 7-derivative of 7-ACA (TzA-7-ACA) with a heterocyclic thiol (MMTD) in aqueous solution, the formed product being cefazolin.

20. Claim : 28 (completely)

    A method for downstreaming where ThAH is removed from an aqueous cephalotin solution by extraction with toluene or xylene at pH 3.5-3.9.

21. Claim : 29 (completely)

    A process for enzymatic cleavage of cephamycin C to 7-MACA by enzymatic oxidative deamination by DAO, spontaneous decarboxylation by hydrogen peroxide, and enzymatic hydrolysis by GAC.

## ANNEX TO THE EUROPEAN SEARCH REPORT ON EUROPEAN PATENT APPLICATION NO. EP 02 07 8608

This annex lists the patent family members relating to the patent documents cited in the above-mentioned European search report. The members are as contained in the European Patent Office EDP file on
The European Patent Office is in no way liable for these particulars which are merely given for the purpose of information.

10-06-2003

| Patent document cited in search report | | Publication date | Patent family member(s) | | | Publication date |
|---|---|---|---|---|---|---|
| WO 9920786 | A | 29-04-1999 | NL | 1007302 | C2 | 20-04-1999 |
| | | | AT | 234934 | T | 15-04-2003 |
| | | | AU | 9560198 | A | 10-05-1999 |
| | | | BR | 9813079 | A | 15-08-2000 |
| | | | CN | 1282377 | T | 31-01-2001 |
| | | | EG | 21235 | A | 31-03-2001 |
| | | | EP | 1023454 | A1 | 02-08-2000 |
| | | | WO | 9920786 | A1 | 29-04-1999 |
| | | | TR | 200000991 | T2 | 21-07-2000 |
| | | | US | 6287799 | B1 | 11-09-2001 |
| EP 0458932 | A | 04-12-1991 | ES | 2036149 | A6 | 01-05-1993 |
| | | | US | 5268271 | A | 07-12-1993 |
| | | | AT | 168134 | T | 15-07-1998 |
| | | | AU | 6960291 | A | 18-07-1991 |
| | | | DE | 69032467 | D1 | 13-08-1998 |
| | | | DE | 69032467 | T2 | 14-01-1999 |
| | | | EP | 0458932 | A1 | 04-12-1991 |
| | | | WO | 9109136 | A1 | 27-06-1991 |
| WO 9804732 | A | 05-02-1998 | AU | 727543 | B2 | 14-12-2000 |
| | | | AU | 3726497 | A | 20-02-1998 |
| | | | EP | 0920527 | A1 | 09-06-1999 |
| | | | HU | 0004931 | A2 | 28-05-2001 |
| | | | IL | 126329 | A | 10-02-2002 |
| | | | JP | 2000516215 | T | 05-12-2000 |
| | | | KR | 2000029604 | A | 25-05-2000 |
| | | | WO | 9804732 | A1 | 05-02-1998 |
| | | | US | 6156534 | A | 05-12-2000 |
| | | | US | 5922907 | A | 13-07-1999 |

EPO FORM P0459

For more details about this annex : see Official Journal of the European Patent Office, No. 12/82